# EUROPÄISCHE PATENTSCHRIFT

(11) **EP 3 695 817 B1**
(45) Veröffentlichungstag und Bekanntmachung des Hinweises auf die Patenterteilung: **10.05.2023**
(21) Anmeldenummer: 20155996.0
(22) Anmeldetag: 07.02.2020
(51) Int. Cl.: A61F 9/008

(54) **BEHANDLUNGSVORRICHTUNG ZUR STEUERUNG EINES AUGENCHIRURGISCHEN LASERS**
TREATMENT DEVICE FOR CONTROLLING AN OPHTHALMIC SURGICAL LASER
DISPOSITIF DE TRAITEMENT D'UN LASER CHIRURGICAL OPHTALMOLOGIQUE

(30) Priorität: 15.02.2019 DE 102019103848
(43) Veröffentlichungstag der Anmeldung: 19.08.2020
(73) Patentinhaber: Schwind Eye-Tech-Solutions GmbH, 63801 Kleinostheim (DE)
(72) Erfinder: ARBA MOSQUERA, Samuel, 63739 Aschaffenburg (DE); TRIEFENBACH, Nico, 63814 Mainaschaff (DE); SHRAIKI, Mario, 64372 Ober-Ramstadt (DE)
(74) Vertreter: Hofstetter, Schurack & Partner

(56) Entgegenhaltungen:
- DE-A1-102007 053 283
- US-A1- 2004 243 112
- US-B1- 6 325 792

## Beschreibung

Die vorliegende Erfindung betrifft ein Computerprogramm zur Steuerung eines augenchirurgischen Lasers für die Abtrennung eines Volumenkörpers mit vordefinierten Grenzflächen aus einer menschlichen oder tierischen Hornhaut. Die Erfindung betrifft weiterhin eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption und mindestens einer Steuereinrichtung für den oder die Laser sowie ein computerlesbares Medium.

Ophthalmologische chirurgische Vorrichtungen und Verfahren unter Einsatz von Laserstrahlung sind bekannt. Die DE 10 2007 053 283 A1 beschreibt eine Vorrichtung und ein Verfahren zur operativen Fehlsichtigkeitskorrektur, wobei durch Einstrahlung von Laserstrahlung Hornhautgewebe getrennt wird. In der US 2004/243112 A1 wird eine ophthalmologische chirurgische Vorrichtung beschrieben, wobei ein augenchirurgischer Laser eingesetzt wird. Die US 6,325,792 B1 beschreibt augenchirurgische Verfahren, wobei mit Hilfe von Laserstrahlung Hornhautgewebe entfernt wird. Vorrichtungen und Verfahren zur Steuerung eines photoablativen ophthalmologischen Lasers sind bekannt. So beschreibt die internationale Patentanmeldung WO 02/22003 eine Vorrichtung zur Bestimmung eines abzutragenden Bereichs von Hornhautgewebe, wobei das Volumen des abzutragenden Gewebes mit Hilfe einer pachymetrischen Vermessung des entsprechenden Bereichs der abzutragenden Hornhaut ermittelt wird. Die ermittelten pachymetrischen Daten dienen dabei zur Herstellung eines Betts innerhalb der Hornhaut zur Aufnahme einer entsprechenden Spenderhornhaut. Auch die US 6,551,306 beschreibt ein Verfahren und eine Vorrichtung zur Kontrolle des Tiefenabtrags auf der Hornhaut. Anhand von pachymetrischen Daten wird während einer Operation ein entsprechender Laser gesteuert und kontrolliert.

Nachteilig an diesen bekannten Verfahren und Vorrichtungen ist jedoch, dass bei den bekannten ablativen Verfahren und Vorrichtungen der gesamte Volumenkörper mittels Laserenergie verdampft wird. Einerseits erfordert dies relativ lange Behandlungszeiten, andererseits erfolgt ein relativ hoher Energieeintrag durch den Laser in die Hornhaut. Insbesondere letzteres kann zu Komplikationen bei der Entfernung des vorbestimmten Volumenkörpers der Hornhaut führen. Diese Nachteile bekannter photoablativer Laser, beispielsweise von Excimer-Lasern, die überwiegen im ultravioletten Bereich emittieren, konnten bisher noch nicht zufriedenstellend gelöst werden.

Es ist daher Aufgabe der vorliegenden Erfindung eine Steuerung eines augenchirurgischen Lasers für die Abtrennung eines Volumenkörpers mit vordefinierten Grenzflächen aus einer menschlichen oder tierischen Hornhaut bereitzustellen, mit denen die Nachteile des Standes der Technik überwunden werden.

Diese Aufgabe wird durch die unabhängigen Patentansprüche gelöst.

Vorteilhafte Ausgestaltungen mit zweckmäßigen Weiterbildungen der Erfindung sind in den jeweiligen Unteransprüchen angegeben, wobei vorteilhafte Ausgestaltungen des Computerprogramms als vorteilhafte Ausgestaltungen der Behandlungsvorrichtung und des computerlesbaren Mediums und umgekehrt anzusehen sind.

Ein erster Aspekt der Erfindung betrifft ein Computerprogramm zur Steuerung eines augenchirurgischen Lasers für die Abtrennung eines Volumenkörpers mit vordefinierten Grenzflächen aus einer menschlichen oder tierischen Hornhaut, umfassend Befehle, die bewirken, dass eine Behandlungsvorrichtung die nachfolgenden beschriebenen Verfahrensschritte durchführt. Das erfindungsgemäße Computerprogramm umfasst dabei ein Steuern des Lasers mittels einer Steuereinrichtung derart, dass dieser gepulste Laserpulse in einem vordefinierten Muster in die Hornhaut abgibt, wobei die Grenzflächen des abzutrennenden Volumenkörpers durch das vordefinierte Muster und eine Oberfläche der Hornhaut definiert sind und die in der Hornhaut liegenden Grenzflächen mittels Photodisruption erzeugt werden, und wobei die Grenzfläche im Wesentlichen quer zu der optischen Achse des Auges unmittelbar oberhalb, oder innerhalb der Bowman-Membran erzeugt wird. Dadurch, dass der abzutrennende Volumenkörper lediglich durch die Grenzflächen beschrieben und definiert ist und diese Grenzflächen mittels Photodisruption erzeugt werden, kann auf einen vollflächigen beziehungsweise vollvolumigen Abtrag des Volumenkörpers verzichtet werden. Es werden lediglich die Grenzflächen mittels Photodisruption erzeugt, sodass anschließend der vordefinierte Volumenkörper aus der Hornhaut entnommen werden kann. Unter dem Begriff "Grenzflächen" ist auch zu verstehen, dass der Volumenkörper gegebenfalls mittels einer einzigen in der Hornhaut liegenden Grenzfläche und der Oberfläche der Hornhaut definiert und abgetrennt werden kann. Durch das erfindungsgemäße Computerprogramm wird einerseits die Behandlungsdauer für die Abtrennung des Volumenkörpers verkürzt, andererseits wird auch der Energieeintrag in die Hornhaut des Patienten signifikant reduziert. Komplikationen, die sich insbesondere durch einen erhöhten Energieeintrag in die Hornhaut ergeben könnten, werden zuverlässig verhindert.

Erfindungsgemäß ist die Oberfläche der Hornhaut dabei eine mittels Abtragen oder Verschieben einer obersten Hornhautschicht und/oder mittels Herstellung einer Hornhautklappe künstlich erzeugte Oberfläche des Auges. Damit ist das erfindungsgemäße Computerprogramm für eine Vielzahl von Verfahren bei der Korrektur von Fehlsichtigkeiten des Auges verwendbar. Insbesondere bei der photorefraktiven Keratektomie (PRK), bei der Laser-epithelialen Keratomileusis (LASIK), der epithelialen Laser-in-situ-Keratomileusis (Epi-LASIK) oder der transepithelialen photorefraktiven Keratektomie (Trans-PRK) kann das erfindungsgemäße Computerprogramm zur Steuerung eines augenchirurgischen Lasers verwendet werden. Es handelt sich dabei um ein Verfahren, bei dem ein Gewebeabtrag unter anderem an einer künstlich erzeugten Hornhautoberfläche stattfindet. Im Gegensatz zu dem bekannten Verfahren zur Gewebeabtragung der künstlichen Hornhautoberfläche wird bei dem erfindungsgemäßen Computerprogramm der Laser jedoch derart gesteuert, dass keine vollflächige Abtragung eines vordefinierten Volumenkörpers der Hornhaut erfolgt, sondern der Volumenkörper durch die genannten Grenzflächen definiert wird und die in der Hornhaut liegenden Grenzflächen mittels Photodisruption erzeugt werden.

Dabei besteht die Möglichkeit, dass der Laser derart gesteuert wird, dass das vordefinierte Muster ausgehend von einer von der Oberfläche der Hornhaut beabstandeten Grenzfläche des Volumenkörpers in Richtung der Oberfläche der Hornhaut abgearbeitet wird. Es besteht aber auch die Möglichkeit, dass der Laser derart gesteuert wird, dass das vordefinierte Muster ausgehend von der Oberfläche der Hornhaut in Richtung einer von der Oberfläche der Hornhaut beabstandeten Grenzfläche des Volumenkörpers abgearbeitet wird. Vorteilhafterweise kann entsprechend den topografischen und/oder pachymetrischen Ausgestaltungen der zu behandelnden Hornhaut entschieden werden, in welcher Reihenfolge die Grenzflächen des abzutrennenden Volumenkörpers abgearbeitet werden.

In weiteren vorteilhaften Ausgestaltungen des erfindungsgemäßen Computerprogramms verläuft die von der Oberfläche der Hornhaut beabstandete Grenzfläche im Wesentlichen quer zu einer optischen Achse des Auges. Unter dem Begriff "quer" ist dabei nicht zu verstehen, dass diese Grenzfläche immer senkrecht zur optischen Achse des Auges verlaufen muss. Vielmehr ist unter dem Begriff "quer" zu verstehen, dass die entsprechende Grenzfläche in unterschiedlichsten Winkeln auf die optische Achse auftreffen kann. So kann die Grenzfläche, die im Wesentlichen quer zur optischen Achse des Auges verläuft, in einem Winkel zwischen 45° und 135° an der optischen Achse anliegen. Des Weiteren kann diese Grenzfläche zumindest teilweise gerade und/oder gekrümmt und/oder wellenartig und/oder gezackt und/oder glatt ausgebildet sein. Auch andere topografische Ausgestaltungen der Grenzfläche quer zur optischen Achse des Auges wie auch den an diese Grenzfläche anschließenden seitlichen Grenzflächen, welche ebenfalls als Grenzflächen zur Definition des Volumenkörpers dienen, sind denkbar.

In weiteren vorteilhaften Ausgestaltungen des erfindungsgemäßen Computerprogramms wird der Laser derart gesteuert, dass das vordefinierte Muster zumindest teilweise kreis- und/oder spiralförmig abgetragen wird. Dabei kann der Start der Photodisruption durch die einzelnen Laserpulse im Zentrum der jeweiligen Grenzfläche oder auch am Rand der jeweiligen Grenzfläche erfolgen.

In weiteren vorteilhaften Ausgestaltungen des erfindungsgemäßen Computerprogramms ist das vordefinierte Muster anhand eines oder mehrerer Steuerdatensätze definiert, wobei der oder die Steuerdatensätze Steuerdaten zur Positionierung und/oder zur Fokussierung einzelner Laserpulse in der Hornhaut umfassen. Die Ermittlung der Steuerdatensätze ist bekannt und ergeben sich insbesondere aus der topografischen und/oder pachymetrischen Vermessung der zu behandelnden Hornhaut sowie der Art und des Umfangs der zu korrigierenden Fehlsichtigkeit.

Erfindungsgemäß ist im Computerprogramms der abzutrennende Volumenkörper derart ausgebildet, dass durch die Entfernung des Volumenkörpers eine refraktive Korrektur des Auges erfolgt. Die Grenzfläche, die im Wesentlichen quer zu der optischen Achse des Auges liegt, wird unmittelbar oberhalb, oder innerhalb der Bowman-Membran erzeugt. Bei den genannten Fehlsichtigkeiten des Auges kann es sich um Myopie, Hyperopie, Presbyopie, Astigmatismus oder auch andere Fehlsichtigkeiten des Auges handeln.

In weiteren vorteilhaften Ausgestaltungen des erfindungsgemäßen Computerprogramms ist vorgesehen, dass der Laser Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abgibt. Derartige Laser werden bereits für photodisruptive Verfahren in der Augenchirurgie verwendet. So beschreibt beispielsweise die EP 2 211 803 B1 einen entsprechenden Femtosekundenlaser, der zur Herstellung eines so genannten Lentikels, das heißt eines linsenartigen Volumenkörpers, innerhalb der Hornhaut verwendet wird. Das so hergestellte Lentikel wird anschließend über einen Schnitt in der Hornhaut aus dieser entnommen. Die Verwendung derartiger photodisruptiver Laser anstelle von ablativ wirkenden Lasern bei der photorefraktiven Keratektomie, das heißt bei Verfahren die einen Abtrag der Hornhautoberfläche zur Folge haben, ist jedoch neu und aus dem Stand der Technik nicht bekannt. Die Verwendung von photodisruptiven Lasern bei dem erfindungsgemäßen Computerprogramm weist zudem den Vorteil auf, dass die Bestrahlung der Hornhaut nicht in einem Wellenlängenbereich unter 300 nm erfolgen muss. Dieser Bereich wird in der Lasertechnik unter dem Begriff "tiefes Ultraviolett" subsumiert. Dadurch wird vorteilhafterweise vermieden, dass durch diese sehr kurzwelligen und energiereichen Strahlen eine unbeabsichtigte Schädigung der Hornhaut erfolgt. Photodisruptive Laser der hier verwendeten Art bringen üblicherweise gepulste Laserstrahlung mit einer Pulsdauer zwischen 1 fs und 1 ns in das Hornhautgewebe ein. Dadurch kann die für den optischen Durchbruch notwendige Leistungsdichte des jeweiligen Laserpulses räumlich eng begrenzt werden, so dass eine hohe Schnittgenauigkeit bei der Erzeugung der Grenzfläche(n) gewährleistet ist.

Ein zweiter Aspekt der vorliegenden Erfindung betrifft eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption und mindestens einer Steuereinrichtung für den oder die Laser, die ausgebildet ist, die Schritte des Computerprogramms gemäß dem ersten Aspekt der Erfindung auszuführen. Die erfindungsgemäße Behandlungsvorrichtung ermöglicht es, dass die bei der Verwendung üblicher ablativer Behandlungsvorrichtungen auftretenden Nachteile, nämlich relativ lange Behandlungszeiten und ein relativ hoher Energieeintrag durch den Laser in die Hornhaut, zuverlässig vermieden werden. Diese Vorteile werden insbesondere durch die Ausbildung des augenchirurgischen Lasers als photodisruptiver Laser erzielt.

Dabei ist der Laser geeignet, Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

In weiteren vorteilhaften Ausgestaltungen der erfindungsgemäßen Behandlungsvorrichtung umfasst die Steuereinrichtung mindestens eine Speichereinrichtung zur zumindest temporären Speicherung von mindestens einem Steuerdatensatz, wobei der oder die Steuerdatensätze Steuerdaten zur Positionierung und/oder zur Fokussierung einzelner Laserpulse in der Hornhaut umfassen; und mindestens eine Strahleneinrichtung zur Strahlführung und/oder Strahlformung und/oder Strahlablenkung und/oder Strahlfokussierung eines Laserstrahls des Lasers aufweist. Die genannten Steuerdatensätze werden dabei üblicherweise anhand einer gemessenen Topografie und/oder Pachymetrie der zu behandelnden Hornhaut und der Art der zu korrigierenden Fehlsichtigkeit des Auges erzeugt.

Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Ein weiterer Aspekt der Erfindung betrifft ein computerlesbares Medium, auf dem das Computerprogramm gespeichert ist. Weitere Merkmale und deren Vorteile sind den Beschreibungen des ersten und zweiten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Weitere Merkmale und deren Vorteile sind den Beschreibungen des ersten und zweiten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung einer erfindungsgemäßen Behandlungsvorrichtung;
- Fig. 2a: eine Prinzipdarstellung der Erzeugung einer ersten Grenzfläche gemäß einer ersten Ausführungsform des erfindungsgemäßen Computerprogramms;
- Fig. 2b: eine Prinzipdarstellung der Erzeugung eines abzutrennenden Volumenkörpers gemäß der ersten Ausführungsform des erfindungsgemäßen Computerprogramms; und
- Fig. 3: eine Prinzipdarstellung der Erzeugung eines abzutrennenden Volumenkörpers gemäß einer zweiten Ausführungsform des erfindungsgemäßen Computerprogramms.

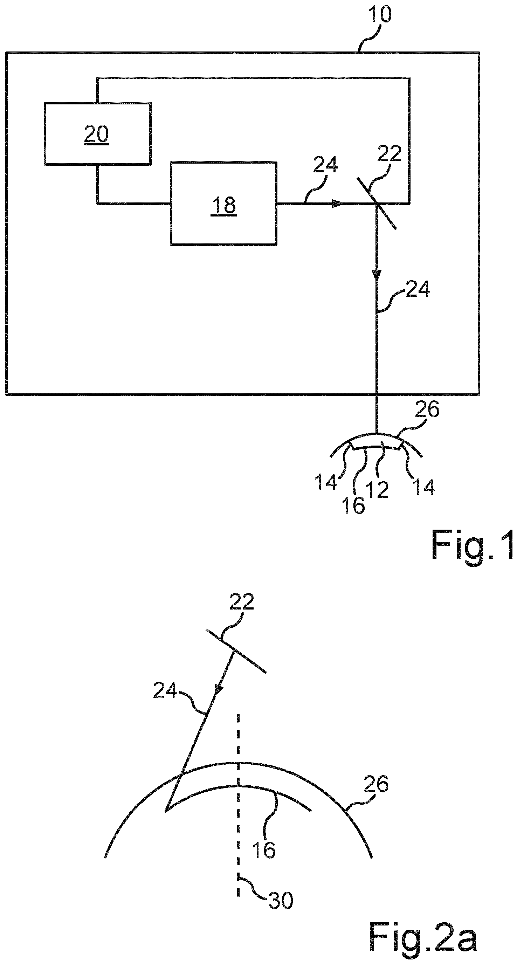

Fig. 1 zeigt eine schematische Darstellung einer Behandlungsvorrichtung 10 mit einem augenchirurgischen Laser 18 für die Abtrennung eines vordefinierten Hornhautvolumens beziehungsweise Volumenkörpers 12 mit vordefinierten Grenzflächen 14, 16 einer Hornhaut eines menschlichen oder tierischen Auges mittels Photodisruption. Man erkennt, dass neben dem Laser 18 eine Steuereinrichtung 20 für den Laser 18 ausgebildet ist, sodass dieser gepulste Laserpulse in einem vordefinierten Muster in die Hornhaut abgibt, wobei die Grenzflächen 14, 16 des abzutrennenden Volumenkörpers 12 durch das vordefinierte Muster und eine Oberfläche 26 der Hornhaut definiert sind und die in der Hornhaut liegenden Grenzflächen 14, 16 mittels Photodisruption erzeugt werden.

Des Weiteren erkennt man, dass der durch den Laser 18 erzeugte Laserstrahl 24 mittels einer Strahleinrichtung 22, nämlich einer Strahlablenkungsvorrichtung, wie zum Beispiel einem Scanner, in Richtung der Oberfläche 26 der Hornhaut abgelenkt wird. Die Strahlablenkvorrichtung 22 wird ebenfalls durch die Steuereinrichtung 20 gesteuert um das genannte vordefinierte Muster in der Hornhaut zu erzeugen.

Bei dem dargestellten Laser 18 handelt es sich um einen photodisruptiven Laser der ausgebildet ist, Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

Die Steuereinrichtung 20 weist zudem eine Speichereinrichtung (nicht dargestellt) zur zumindest temporären Speicherung von mindestens einem Steuerdatensatz auf, wobei der oder die Steuerdatensätze Steuerdaten zur Positionierung und/oder zur Fokussierung einzelner Laserpulse in der Hornhaut umfassen. Die Positionsdaten und/oder Fokussierungsdaten der einzelnen Laserpulse werden anhand einer zuvor gemessenen Topografie und/oder Pachymetrie der Hornhaut und der zu korrigierenden Fehlsichtigkeit des Auges erzeugt.

Fig. 2a zeigt eine Prinzipdarstellung der Erzeugung einer ersten Grenzfläche 16 gemäß einer ersten Ausführungsform des vorliegenden Computerprogramms. Man erkennt, dass mittels des gepulsten Laserstrahls 24, der über die Strahlablenkvorrichtung 22 in Richtung der Hornhaut beziehungsweise der Oberfläche 26 der Hornhaut gelenkt wird, eine erste Grenzfläche 16, die ungefähr quer zu einer optischen Achse 30 des Auges verläuft, innerhalb der Hornhaut erzeugt wird. Insbesondere wird die Grenzfläche 16 innerhalb des so genannten Stromas der Hornhaut erzeugt. Dabei kann die Grenzfläche 16 beispielsweise unmittelbar unter der so genannten Bowmanschen Membran liegen.

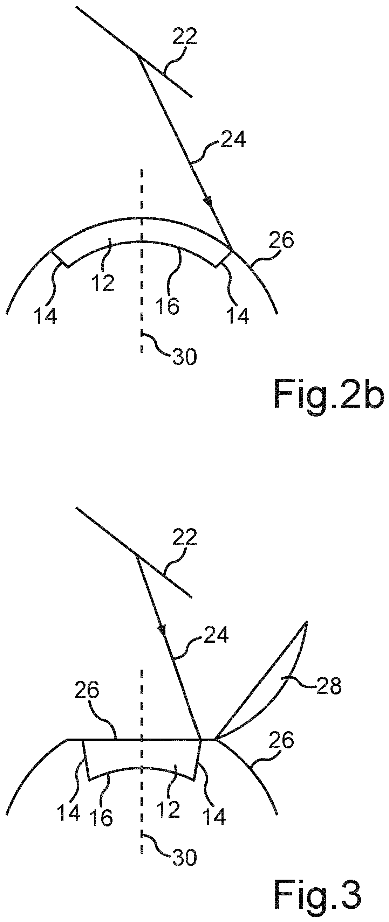

Man erkennt, dass die Grenzfläche 16 ungefähr quer zur optischen Achse 30 liegt, wobei die Grenzfläche 16 gekrümmt ausgebildet ist. Die Ausgestaltung der Grenzfläche 16, wie auch der beiden seitlichen Grenzflächen 14 (vergleiche Fig. 2b) richtet sich nach der Art der zu korrigierenden Fehlsichtigkeit des Auges. Dabei kann insbesondere die Ausgestaltung der Grenzfläche 16 zur Korrektur der Fehlsichtigkeit des Auges beitragen.

Fig. 2b zeigt eine Prinzipdarstellung der Erzeugung des abzutrennenden Volumenkörpers 12 gemäß der in Fig. 2a beschriebenen ersten Ausführungsform des vorliegenden Computerprogramms. Man erkennt, dass die Strahlablenkvorrichtung 22 den Laserstrahl 24 derart steuert, dass die seitlichen Grenzflächen 14 ausgehend von der Grenzfläche 16 in Richtung der Oberfläche 26 der Hornhaut abgearbeitet werden. Dadurch entstehen seitliche Schnitte, die einerseits die Enden der Grenzfläche 16 und andererseits die Oberfläche 26 durchdringen um insgesamt den Volumenkörper 12 auszubilden, sodass dieser aus der Hornhaut abgetrennt werden kann.

Fig. 3 zeigt eine Prinzipdarstellung der Erzeugung eines abzutrennenden Volumenkörpers 12 gemäß einer zweiten Ausführungsform des vorliegenden Computerprogramms. Man erkennt, dass vor einer Erzeugung der Grenzfläche 16 wie auch der Erzeugung der Grenzflächen 14 eine so genannte Hornhautklappe 28 erzeugt wird. Die Hornhautklappe 28 kann dabei mittels einer mechanischen Schneidevorrichtung oder auch mittels eines Lasers erzeugt werden. Zur Ausbildung der Hornhautklappe 28 kann auch der Laser 18 verwendet werden. Durch ein Zurückklappen der Hornhautklappe 28 gibt diese eine Oberfläche 26 der Hornhaut frei, aus der wiederum der vordefinierte Volumenkörper 12 mit den Grenzflächen 14, 16 abgetrennt werden kann. Nach der Herausnahme des Volumenkörpers 12 aus der Hornhaut wird die Hornhautklappe 28 wieder zurückgeklappt und verschließt somit den durch die Herausnahme des Volumenkörpers 12 erzeugten Hohlraum innerhalb der Hornhaut.

Bezüglich der Erklärung der weiteren Merkmale der Fig. 3 verweisen wir auf die Figuren 2a und 2b, wobei identische Merkmale mit gleichen Bezugszeichen gekennzeichnet sind.

## Patentansprüche

1. Computerprogramm zur Steuerung eines augenchirurgischen Lasers (18) für die Abtrennung eines Volumenkörpers (12) mit vordefinierten Grenzflächen (14, 16) aus einer menschlichen oder tierischen Hornhaut, umfassend Befehle, die bewirken, dass eine Behandlungsvorrichtung (10) den Laser (18) mittels einer Steuereinrichtung (20) derart steuert, dass dieser gepulste Laserpulse in einem vordefinierten Muster in die Hornhaut abgibt, wobei die Grenzflächen (14, 16) des abzutrennenden Volumenkörpers (12) durch das vordefinierte Muster und eine Oberfläche (26) der Hornhaut definiert sind und die in der Hornhaut liegenden Grenzflächen (14, 16) mittels Photodisruption erzeugt werden, und wobei die Grenzfläche (16) im Wesentlichen quer zu der optischen Achse (30) des Auges unmittelbar oberhalb oder innerhalb der Bowman-Membran erzeugt wird, und wobei die Oberfläche (26) der Hornhaut eine mittels Abtragen oder Verschieben einer obersten Hornhautschicht und/oder mittels Herstellung einer Hornhautklappe (28) künstlich erzeugte Oberfläche des Auges ist.

2. Computerprogramm nach Anspruch 1, **dadurch gekennzeichnet, dass** der Laser (18) derart gesteuert wird, dass das vordefinierte Muster ausgehend von einer von der Oberfläche (26) der Hornhaut beabstandeten Grenzfläche (16) des Volumenkörpers (12) in Richtung der Oberfläche (26) der Hornhaut abgearbeitet wird.

3. Computerprogramm nach Anspruch 1, **dadurch gekennzeichnet, dass** der Laser (18) derart gesteuert wird, dass das vordefinierte Muster ausgehend von der Oberfläche (26) der Hornhaut in Richtung einer von der Oberfläche (26) der Hornhaut beabstandeten Grenzfläche (16) des Volumenkörpers (12) abgearbeitet wird.

4. Computerprogramm nach einem der vorhergehenden Ansprüche, **dadurch gekennzeichnet, dass** die quer zu der optischen Achse (30) des Auges verlaufende Grenzfläche (16) zumindest teilweise gerade und/oder gekrümmt und/oder wellenartig und/oder gezackt und/oder glatt ausgebildet ist.

5. Computerprogramm nach einem der vorhergehenden Ansprüche, **dadurch gekennzeichnet, dass** die im Wesentlichen quer zu der optischen Achse (30) des Auges verlaufende Grenzfläche (16) in einem Winkel von zwischen 45° und 135° an der optischen Achse (30) anliegt.

6. Computerprogramm nach einem der vorhergehenden Ansprüche, **dadurch gekennzeichnet, dass** der Laser (18) derart gesteuert wird, dass das vordefinierte Muster zumindest teilweise kreis- und/oder spiralförmig abgetragen wird.

7. Computerprogramm nach einem der vorhergehenden Ansprüche, **dadurch gekennzeichnet, dass** das vordefinierte Muster anhand eines oder mehrerer Steuerdatensätze definiert ist, wobei der oder die Steuerdatensätze Steuerdaten zur Positionierung und/oder zur Fokussierung einzelner Laserpulse in der Hornhaut umfassen.

8. Computerprogramm nach einem der vorhergehenden Ansprüche, **dadurch gekennzeichnet, dass** durch das Computerprogramm der abzutrennende Volumenkörper (12) derart ausgebildet wird, dass durch die Entfernung des Volumenkörpers (12) eine Korrektur von Fehlsichtigkeiten des Auges erfolgt.

9. Computerprogramm nach einem der vorhergehenden Ansprüche, **dadurch gekennzeichnet, dass** durch das Computerprogramm der Laser (18) Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abgibt.

10. Behandlungsvorrichtung (10) mit mindestens einem augenchirurgischen Laser (18) für die Abtrennung eines vordefinierten Hornhautvolumens (12) mit vordefinierten Grenzflächen (14, 16) eines menschlichen oder tierischen Auges mittels Photodisruption und mindestens einer Steuereinrichtung (20) für den oder die Laser (18), die ausgebildet ist, die Schritte des Computerprogramms nach einem der Ansprüche 1 bis 9 auszuführen.

11. Behandlungsvorrichtung nach Anspruch 10, **dadurch gekennzeichnet, dass** der Laser (18) geeignet ist Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

12. Behandlungsvorrichtung nach Anspruch 10 oder 11, **dadurch gekennzeichnet, dass** die Steuereinrichtung (20)
- mindestens eine Speichereinrichtung zur zumindest temporären Speicherung von mindestens einem Steuerdatensatz aufweist, wobei der oder die Steuerdatensätze Steuerdaten zur Positionierung und/oder zur Fokussierung einzelner Laserpulse in der Hornhaut umfassen; und
- mindestens eine Strahleinrichtung (22) zur Strahlführung und/oder Strahlformung und/oder Strahlablenkung und/oder Strahlfokussierung eines Laserstrahls (24) des Lasers (18) umfasst.

13. Computerlesbares Medium, auf dem das Computerprogramm nach einem der Ansprüche 1 bis 9 gespeichert ist.

## Claims

1. A computer program for controlling an eye surgical laser (18) for the separation of a volume body (12) with predefined interfaces (14, 16) from a human or animal cornea, comprising commands, which cause a treatment device (10) to control the laser (18) by means of a control device (20) such that it emits pulsed laser pulses in a predefined pattern into the cornea, wherein the interfaces (14, 16) of the volume body (12) to be separated are defined by the predefined pattern and a surface (26) of the cornea and the interfaces (14, 16) located in the cornea are generated by means of photodisruption, and wherein the interface (16) is generated substantially transversely to the optical axis (30) of the eye immediately above or within the Bowman's membrane, and wherein the surface (26) of the cornea is a surface of the eye artificially generated by means of ablation or displacement of an uppermost corneal layer and/or by means of production of a corneal flap (28).

2. The computer program according to claim 1, **characterized in that** the laser (18) is controlled such that the predefined pattern is processed starting from an interface (16) of the volume body (12) spaced from the surface (26) of the cornea in the direction of the surface (26) of the cornea.

3. The computer program according to claim 1, **characterized in that** the laser (18) is controlled such that the predefined pattern is processed starting from the surface (26) of the cornea in the direction of an interface (16) of the volume body (12) spaced from the surface (26) of the cornea.

4. The computer program according to any one of the preceding claims, **characterized in that** the interface (16) that extends substantially transversely to the optical axis (30) is formed at least partially straight and/or curved and/or wave-like and/or serrated and/or smooth transversely to the optical axis (30) of the eye.

5. The computer program according to any one of the preceding claims, **characterized in that** the interface (16) lies on the optical axis (30) at an angle between 45° and 135° substantially transversely to the optical axis (30) of the eye.

6. The computer program according to any one of the preceding claims, **characterized in that** the laser (18) is controlled such that the predefined pattern is at least partially circularly and/or spirally ablated.

7. The computer program according to any one of the preceding claims, **characterized in that** the predefined pattern is defined based on one or more control datasets, wherein the control dataset or datasets include control data for positioning and/or for focusing individual laser pulses in the cornea.

8. The computer program according to any one of the preceding claims, **characterized in that** by means of the computer program the volume body (12) to be separated is formed such that a correction of visual disorders of the eye is effected by the removal of the volume body (12).

9. The computer program according to any one of the preceding claims, **characterized in that** by the computer program the laser (18) emits laser pulses in a wavelength range between 300 nm and 1400 nm, preferably between 900 nm and 1200 nm, at a respective pulse duration between 1 fs and 1 ns, preferably between 10 fs and 10 ps, and a repetition frequency of greater than 10 KHz, preferably between 100 KHz and 100 MHz.

10. A treatment device (10) with at least one eye surgical laser (18) for the separation of a predefined corneal volume (12) with predefined interfaces (14, 16) of a human or animal eye by means of photodisruption and at least one control device (20) for the laser or the lasers (18), which is formed to execute the steps of the computer program according to any one of claims 1 to 9.

11. The treatment device according to claim 10, **characterized in that** the laser (18) is suitable to emit laser pulses in a wavelength range between 300 nm and 1400 nm, preferably between 900 nm and 1200 nm, at a respective pulse duration between 1 fs and 1 ns, preferably between 10 fs and 10 ps, and a repetition frequency of greater than 10 KHz, preferably between 100 KHz and 100 MHz.

12. The treatment device according to claim 10 or 11, **characterized in that** the control device (20)
- comprises at least one storage device for the at least temporary storage of at least one control dataset, wherein the control dataset or datasets include control data for positioning and/or for focusing individual laser pulses in the cornea; and
- comprises at least one beam device (22) for beam guidance and/or beam shaping and/or beam deflection and/or beam focusing of a laser beam (24) of the laser (18).

13. A computer-readable medium, on which the computer program according to any of claims 1 to 9 is stored.

## Revendications

1. Programme informatique de commande d'un laser chirurgical ophtalmique (18) pour la séparation d'un corps volumique (12) avec des interfaces prédéfinies (14, 16) d'une cornée humaine ou animale, comprenant des instructions qui amènent un dispositif de traitement (10) à commande le laser (18) au moyen d'un dispositif de commande (20), de telle sorte que celui-ci émet des impulsions laser pulsées selon un modèle prédéfini dans la cornée, dans lequel les interfaces (14, 16) du corps volumique (12) à séparer sont définies par le modèle prédéfini et une surface (26) de la cornée et les interfaces (14, 16) situées dans la cornée sont générées au moyen d'une photodisruption, et dans lequel l'interface (16) est générée de manière sensiblement transversale à l'axe optique (30) de l'oeil directement au-dessus ou à l'intérieur de la membrane de Bowman, et dans lequel l'interface (26) de la cornée est une surface générée artificiellement au moyen d'une ablation ou d'un déplacement d'une couche de cornée supérieure et/ou au moyen de la production d'un volet cornéen (28).

2. Programme informatique selon la revendication 1, **caractérisé en ce que** le laser (18) est commandé de telle sorte que le modèle prédéfini est traité en partant d'une interface (16) du corps volumique (12) espacée de la surface (26) de la cornée, en direction de la surface (26) de la cornée.

3. Programme informatique selon la revendication 1, **caractérisé en ce que** le laser (18) est commandé de telle sorte que le modèle prédéfini est exécuté en partant de la surface (26) de la cornée, en direction d'une interface (16) du corps volumique (12) espacée de la surface (26) de la cornée.

4. Programme informatique selon l'une des revendications précédentes, **caractérisé en ce que** l'interface (16) s'étendant transversalement à l'axe optique (30) de l'oeil est formée au moins partiellement droite et/ou incurvée et/ou ondulée et/ou dentelée et/ou lisse.

5. Programme informatique selon l'une des revendications précédentes, **caractérisé en ce que** l'interface (16) s'étendant de manière sensiblement transversale à l'axe optique (30) de l'oeil se situe sur l'axe optique (30) à un angle compris entre 45° et 135°.

6. Programme informatique selon l'une des revendications précédentes, **caractérisé en ce que** le laser (18) est commandé de telle sorte le modèle prédéfini est enlevé de manière au moins partiellement circulaire et/ou en spirale.

7. Programme informatique selon l'une des revendications précédentes, **caractérisé en ce que** le modèle prédéfini est défini au moyen d'un ou plusieurs ensembles de données de commande, l'ensemble ou les ensembles de données de commande comprenant des données de commande pour le positionnement et/ou la focalisation d'impulsions laser individuelles dans la cornée.

8. Programme informatique selon l'une des revendications précédentes, **caractérisé en ce que** le corps volumique (12) à séparer est formé par le programme informatique de telle sorte qu'une correction de troubles visuels de l'oeil est réalisée par l'ablation du corps volumique (12).

9. Programme informatique selon l'une des revendications précédentes, **caractérisé en ce que** le laser (18) émet des impulsions laser, via le programme informatique, dans une gamme de longueurs d'onde entre 300 nm et 1 400 nm, de préférence entre 900 nm et 1 200 nm, à une durée d'impulsion respective entre 1 fs et 1 ns, de préférence entre 10 fs et 10 ps, et une fréquence de répétition supérieure à 10 kHz, de préférence entre 100 kHz et 100 MHz.

10. Dispositif de traitement (10) avec au moins un laser chirurgical ophtalmique (18) pour la séparation d'un volume de cornée prédéfini (12) avec des interfaces (14, 16) prédéfinies d'un oeil humain ou animal au moyen d'une photodisruption, et au moins un dispositif de commande (20) pour le ou les lasers (18), qui est conçu pour exécuter les étapes du programme informatique selon l'une des revendications 1 à 9.

11. Dispositif de traitement selon la revendication 10, **caractérisé en ce que** le laser (18) est adapté pour émettre des impulsions laser dans une gamme de longueurs d'onde entre 300 nm et 1 400 nm, de préférence entre 900 nm et 1 200 nm, à une durée d'impulsion respective entre 1 fs et 1 ns, de préférence entre 10 fs et 10 ps, et une fréquence de répétition supérieure à 10 kHz, de préférence entre 100 kHz et 100 MHz.

12. Dispositif de traitement selon la revendication 10 ou 11, **caractérisé en ce que** le dispositif de commande (20) comprend
- au moins un dispositif de stockage pour le stockage au moins temporaire d'au moins un ensemble de données de commande, l'ensemble ou les ensembles de données de commande comprenant des données de commande pour le positionnement et/ou la focalisation d'impulsions laser individuelles dans la cornée ; et
- au moins un dispositif à faisceau (22) pour le guidage de faisceau et/ou la conformation de faisceau et/ou la déviation de faisceau et/ou la focalisation de faisceau d'un faisceau laser (24) du laser (18).

13. Support lisible par ordinateur sur lequel est stocké le programme informatique selon l'une des revendications 1 à 9.
